# EUROPEAN PATENT APPLICATION

(11) **EP 1 262 541 A1**
(43) Date of publication of application: **04.12.2002**
(21) Application number: 01202013.7
(22) Date of filing: 28.05.2001
(51) Int. Cl.: C12N 1/00, C12N 15/09, C12P 13/02, C12P 17/18, C12P 7/44, A61K 35/66, A23L 1/302, A23L 1/305

(54) **Production of bioavailable folic acid**

(71) Applicant: Stichting Top-Instituut Voedselwetenschappen, 6700 AN Wageningen (NL)
(72) Inventor: Sybesma, Wilbert Feike Henricus, 6708 AE Wageningen (NL); Hugenholtz, Jeroen, 6721 MR Bennekom (NL); Mierau, Igor, 7371 GE Loenen (NL); Starrenburg, Maria Johanna Catharina, 6721 KA Bennekom (NL); Kleerebezem, Michiel, 6716 GG Ede (NL); De Vos, Willem Meindert, 6721 SJ Bennekom (NL)
(74) Representative: Jorritsma, Ruurd

(57) **Abstract**

The invention provides a process of producing bio-available folate, i.e. folic acid having an increased proportion of monoglutamyl folate and a decreased proportion of polyglutamyl folate, by culturing micro-organisms containing an active heterologous or homologous polyglutamyl hydrolase activity or containing increased activities of folate biosynthesis enzymes. The genes encoding the polyglutamyl hydrolase and the folate biosynthesis enzymes may be of various origin, e.g. from rodents or other mammals including man. Also provided is a foodstuff, especially a dairy product containing such monoglutamyl folate-producing micro-organisms.

## Description

The present invention relates to the increased production of folate in general and, specifically, bioavailable folate, i.e. folate which can be readily absorbed the mammalian gastrointestinal tract, using genetically altered micro-organisms.

### Background

Folate (N-[4-{[(2-amino-1,4-dihydro-4-oxo-6-pteridinyl)methyl]amino}benzoyl]-L-glutamic acid; vitamin B₁₁; vitamin M) is a hematopoietic vitamin, which is increasingly recognised as playing an important role in human and animal health and healing. Folate cannot be produced by mammals. The main sources of folate are plants, especially spinach and other green-leaved vegetables, grasses, yeasts and other micro-organisms, and, indirectly, animal organs (kidney, liver). Supplementation of folate currently occurs by administration as such or in vitamin preparations. However, administration of such vitamins outside the regular diet is expensive and not always accepted.

Folate is a general term for a large number of different folic acid derivatives; they differ in the state of oxidation, one-carbon substitution of the pteridine ring and in the number of glutamate residues. These differences are associated with different physicochemical properties, which, together with certain food constituents, may influence folate bioavailability. One important factor of folate bioavailability is folate stability. Exposure to oxygen, heat and, most importantly, the acidic-peptic environment of the stomach increases folate instability. The presence in foods of antioxidants such as ascorbic acid and reduced thiols protects against this instability.

Another factor that influences folate bioavailability is the presence of polyglutamyl residues on folates. The available information indicates that bioavailability of monoglutamyl folate is higher than the bioavailability of polyglutamyl folate. Polyglutamyl folates must be hydrolysed to the respective monoglutamyl derivatives before they are absorbed by the intestine. This conversion is catalysed by intestinal hydrolases. These enzymes, however, are susceptible to inhibition by the constituents found in some foods. In addition, the activity of these enzymes could also be influenced by the glutamate chain length (Gregory 1989). It can be concluded that the occurrence of folate as the monoglutamyl derivative increases the bioavailability of folates.

Folate is present in food products such as meat, vegetables and dairy products. The amount of monoglutamyl folates, and hence the bioavailability of folate, varies considerably between food products, as was analysed for egg yolk (72% monoglutamyl folates, MGF), cow liver (56% MGF), orange juice (21% MGF), cabbage (6% MGF), lima beans (5% MGF), and lettuce (less than 1% MGF) (Seyoum and Selhub, 1998).

Intracellularly stored folate in lactic acid bacteria is mainly present in the polyglutamyl folate form. One of the functions of the glutamate tail of polyglutamyl folates in micro-organisms is retention of folate in the cell (Shane and Stokstad, 1975). As a consequence, it is possible that polyglutamyl folates remain inside the bacteria during passage through the gastrointestinal (GI) tract and as such are not available for uptake by the human body. This problem may be overcome by increasing the amount of monoglutamyl folate hence reducing the retention of intracellularly stored folate.

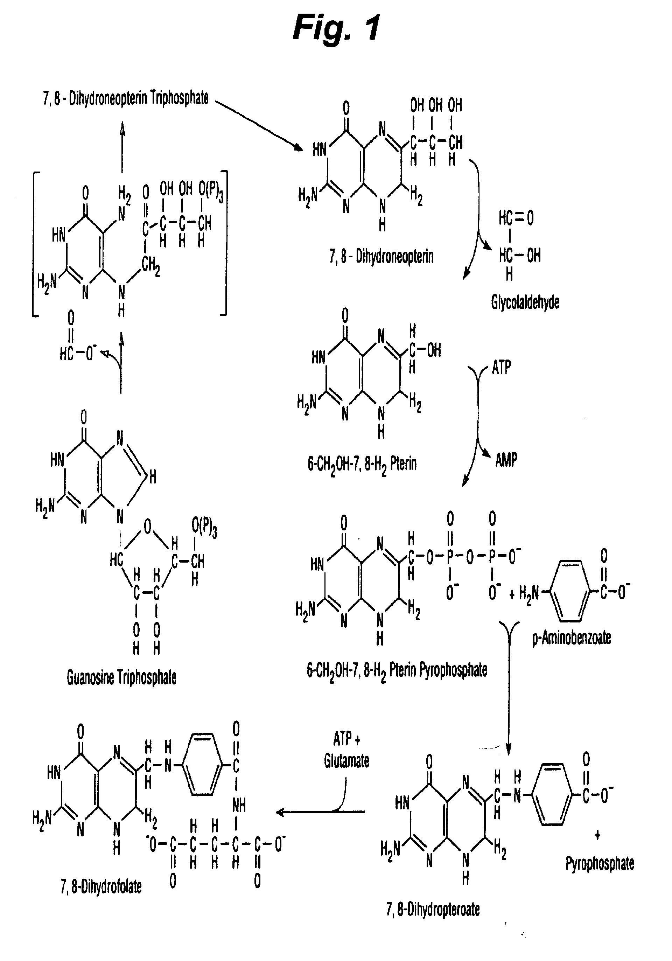

Folate can be synthesised from the precursors GTP, para-aminobenzoic acid and glutamate in a multi-enzyme pathway (see figure 1). In several micro-organisms, including the lactic acid bacterium *Lactococcus lactis,* the genes coding for this pathway - *gch, folC, folP, dhna, hppk and dhfr -* are organised in a gene cluster. GTP cyclohydrolase I *(gch,* EC 3.5.4.16), catalyses the reaction from GTP to dihydroneopterin triphosphate through an intermediate and release of formate. A phosphate residue is then removed presumably by the action of a phosphatase (Pase). Dihydroneopterin aldolase (EC 4.1.2.25) acts on the former reaction product to synthesise glycolaldehyde and 6-hydroxymethyl-7,8-dihydropterin, that is converted to 6-hydroxymethyl-7,8-dihydropterin pyrophosphate by 2-amino-4-hydroxy-6-hydroxymethyldihydropteridine pyrophosphokinase *(hppk,* EC 2.5.1.15). Dihydropteroate synthase (EC 2.7.6.3) couples para-aminobenzoate to produce 7,8-dihydropteroate. Folate synthase (*folC,* EC 6.3.2.17) couples glutamate to dihydropteroate to produce dihydrofolate. After further reduction to tetrahydrofolate, by dihydrofolate reductase (*dhfr,* EC 1.5.1.3), addition of glutamate to the carboxyl group of the side chain of earlier coupled glutamate residues by folyl polyglutamate synthase (*folC*, EC 6.3.2.17) finally produces polyglutamyl tetrahydrofolate (not shown in figure 1).

### Summary of the invention

It has been found now that micro-organisms can be prepared which are capable of producing not only more folate but also folate in a bioavailable form. The invention accordingly provides a recombinant micro-organism, capable of producing polyglutamyl folate, and containing an active heterologous or homologous gamma-glutamyl hydrolase gene, or a genetically altered micro-organism capable of producing not only more folate by overexpression of individual biosynthetic genes, but also relatively lower amounts of polyglutamyl folates, thus favouring excretion of folate into the exterior. The invention further provides expression cassettes comprising a gamma-glutamyl hydrolase gene or a biosynthetic gene such as the GTP cyclohydrolase gene with suitable, preferably microbial, regulatory sequences, and a process for producing folate by culturing micro-organisms producing folate and containing such an expression cassette. The invention also provides foodstuffs, especially dairy products such as yoghurt and other fermented foodstuffs, containing micro-organisms producing high amounts of bioavailable folate.

### Detailed description of the invention

The present invention relates to increasing the production of folate, and specifically, bioavailable folate, i.e. folate which can be readily absorbed by the mammalian gastrointestinal tract. The invention is directed to higher production of various folate derivatives and conversion of various polyglutamyl folate derivatives, as produced by organisms in vivo, to the corresponding monoglutamyl folate derivatives. The main consequences of the invention are threefold:
- Firstly, the invention changes the ratio between poly- and monoglutamyl folate derivatives favouring the monoglutamyl folate derivatives and thus increasing the bioavailability of folate derivatives in general and monoglutamyl folate derivatives in particular.
- Secondly, the invention limits the retention of folate derivatives in the cells of the folate producing host cells enhancing an increased diffusion or transport of folate towards the extracellular environment. As a consequence, the bioavailability of folate increases since intracellularly stored folate is not available for uptake by mammalian gastrointestinal tract.
- Thirdly, the invention enables the cells to produce higher amounts of total folate.

Thus the invention concerns a micro-organism capable of producing higher levels of intracellular folate by overproduction of homologous or heterologous enzymes involved in monoglutamyl folate biosynthesis, or containing an active heterologous glutamyl hydrolase gene. An active glutamyl hydrolase gene is understood to be a gene which, upon expression yields an enzyme capable of deconjugating polyglutamyl folates to monoglutamyl folates.

The folate biosynthetic genes may be any one of the genes in the folate gene cluster, e.g. GTP cyclohydrolase I (gch), dihydroneopterin aldolase (dhna), 2-amino-4-hydroxy-6-hydroxymethyldihydropteridine pyrophosphokinase (hppk), dihydropteroate synthase (dps), folate synthase (folC) and dihydrofolate reductase (dhfr) or a combination thereof, such as gch and hppk. The altered enzyme activity may be the result of altered expression of the enzymes encoded by the genes mentioned above, e.g. by the presence of multiple copies of the homologous or heterologous genes, and/or by the presence of strong promoters for these genes. The altered activity may be enhanced activity or, decreased activity, either one resulting in relative increase of monoglutamyl folate production.

The glutamyl hydrolase may be an endopeptidase, i.e. a glutamyl hydrolase acting on the inside of the polyglutamyl chain resulting in removal of a polyglutamyl chain in a single step. The glutamyl hydrolase may also be an exopeptidase, i.e. an enzyme cleaving off glutamyl residues from the terminus of the polyglutamyl chain resulting in removal of a monoglutamyl residues in repeated steps. Endo-glutamyl hydrolase genes can originate from e.g. rats, whereas exo-glutamyl hydrolase genes are available e.g. from human origin.

The glutamyl hydrolase gene and the folate biosynthetic genes introduced into the micro-organism may the original (unmodified) genes. They may also be modified genes at least containing the region encoding the catalytic domain of the glutamyl hydrolase and the folate biosynthetic enzymes. In particular, the glutamyl hydrolase encoded by the heterologous gene has at least 65% sequence identity, especially at least 75% identity - as determined using the conventional BLAST algorithm - with the amino acid sequences of rat or human gamma-glutamyl hydrolase as described by Yao et al., Proc. Natl. Acad. Sci. USA. 1996, 93(19): 10134-8 and J. Biol. Chem. 1996 271(15): 8525-8, and/or its coding sequence is capable of hybridising under stringent conditions with the nucleotide sequences described by Yao et al.

The term "folate" covers folic acid and any salt, ester and derivative, including methylated derivatives thereof. The term "polyglutamyl folate" refers to folate having at least two contiguous glutamyl residues in its side chain, whereas "monoglutamyl folate" refers to such folate in which a substantial proportion, i.e. at least 25% of the folate molecules, has only one glutamyl group.

The invention thus pertains to intracellular expression of genes encoding gamma-glutamyl hydrolase or folate biosynthesis genes originating from vertebrates, plants, fungi or bacteria in food-grade micro-organisms resulting in intracellular gamma-glutamyl hydrolytic activity. Especially preferred are gamma-glutamyl hydrolase from rat (Yao et al. 1996a), human (Yao et al. 1996b, US 5,801,031), *Arabidopsis thaliana* (Huangpu et al. 1996), soy bean (Huangpu et al. 1996) and from the Gram-positive bacteria *Bacillus subtilis* (Margot et al, 1999), *Bacillus sphaericus* (Hourdou et al, 1992), and *Bacillus* *intermedius* (Leshchinskaya et al, 1997), as well as functionally equivalent hydrolases having at least 65% er even at least 75% sequence identity with these proteins. In more detail the invention is based on the integration of the gene encoding the mentioned gamma-glutamyl hydrolases in a plasmid or in the chromosome of food-grade micro-organisms in such a way that effective expression of the gene occurs, either constitutively or upon induction.

The invention also concerns expression of the genes encoding the gamma-glutamyl hydrolases or the genes encoding folate biosynthesis genes mentioned above in such a way that a fully or partially active enzyme is obtained. The activity of these enzymes results either in deconjugation of polyglutamyl folate derivatives. In this reaction mono- and/or poly-glutamate residues are enzymatically hydrolysed from polyglutamyl folate derivatives creating monoglutamyl folate derivatives, or folate is synthesised in such a way that it contains only a single glutamate residue.

The invention thus allows an increase of the production of overall folate and, specifically, monoglutamyl folate derivatives originating from polyglutamyl folate derivatives after expression of the gene encoding the gamma-glutamyl hydrolases or formed directly, without attachment of a polyglutamyl moiety, by overexpression of folate biosynthetic genes as described above. This limits the retention of poly-glutamyl folate derivatives in the cell enabling an increased active or passive transport of monoglutamyl folate over the cell membrane to the extracellular environment. As a consequence, this results in an increased production and bioavailability of folate derivatives in general and monoglutamyl folate derivatives particular in fermented foods.

As shown in example 1 described below, the intracellular expression and translation of gamma-glutamyl hydrolase from rat or human origin in lactic acid bacteria results in a deconjugation of polyglutamyl folate to monoglutamyl folate, a decrease in retention of intracellular folate, an increase of monoglutamyl folate and an increase of extracellular folate. Thus the products and processes according to the invention lead to increased bioavailability of folate in fermented food. This increase in bioavailability is a result of two phenomena. Firstly, the increase of monoglutamyl folate limits the necessity of activity of natural intestinal hydrolases to deconjugate polyglutamyl folates, which is advantageous because of the susceptibility of the activity of intestinal hydrolases to certain food components. Secondly, the activity of gamma-glutamyl hydrolases expressed in micro-organisms limits the retention of folates in the cell resulting in an increased extracellular folate concentration which is advantageous under conditions when lactic acid bacteria remain intact during passage through the GI tract and hence folate is not released. Moreover, increased export of (monoglutamyl) folate from the cell will increase the overall rate of folate biosynthesis resulting from a reduction of feedback control.

Example 2 shows the effect of overexpression of GTP-cyclohydrolase (EC 3.5.4.16) and of 2-amino-4-hydroxy-6-hydroxymethyldihydropteridine pyrophosphokinase (EC 2.7.6.3) in *Lactococcus lactis*. In *L. lactis,* the gene *gch* encodes a bifunctional protein of these two enzymes. The effect is, not only, an overall increased production of folate in cultures using the recombinant *L. lactis,* but also a specific increase of monoglutamyl folates at the expense of polyglutamyl folates. This can be explained by the relative low activity of the folyl polyglutamate synthase enzyme, encoded by the *folC* gene, which is not high enough to accommodate the higher flux of folate biosynthesis upon overexpression of GTP-cyclohydrolase. The monoglutamyl folates can be excreted more easily by the *L. lactis* cells resulting fermentation broth with, not only, increased levels of folate but also in a more bioavailable form, excreted in the medium as the monoglutamyl form.

Although example 1 concerns gamma-glutamyl hydrolase from rat or human origin and *Lactococcus lactis* strain NZ9000 as the (poly/mono)-glutamyl folate producing lactic acid bacteria, the invention also covers any other gamma-glutamyl hydrolases in general and food-grade gamma-glutamyl hydrolases in particular. Examples of other gamma-glutamyl hydrolases that can be expressed in lactic acid bacteria are from *Arabidopsis*, soy bean or *Bacillus intermedius* origin. The invention also covers the expression of mentioned gamma-glutamyl hydrolases in any other food-grade micro-organism than *L. lactis* strain NZ9000, such as other lactic acid bacteria, yeasts and fungi. In the examples the mature rat or human gamma-glutamyl hydrolase genes were cloned behind a nisin inducible promoter. The invention also covers expression of gamma-glutamyl hydrolases under control of a different inducible promoter, for instance the promoter present in the lactose operon (Van Rooijen et al. 1990, 1992) or a constitutive promoter of, for instance, the *pepN* gene (Tan et al. 1992).

Although example 2 concerns overexpression of the GTP-cyclohydrolase, encoded by homologous *gch* gene in *Lactococcus lactis,* resulting in higher production of folate and a specific increase of monoglutamyl folates versus polyglutamyl folates, the invention also covers overexpression of other homologous and heterologous folate biosynthetic genes in *L. lactis.* In addition, the invention covers the expression of these folate biosynthetic genes in other food-grade micro-organisms than *L. lactis* strain NZ9000, such as other lactic acid bacteria, yeasts and fungi. In example 2 the *gch* gene was cloned behind the nisin inducible promoter. The invention also covers expression of GTP-cyclohydrolase and other folate biosynthetic enzymes under the control of a different inducible promoter, for instance the promoter present I the lactose operon (Van Rooijen et al. 1990, 1992) or a constitutive promoter as found in front of the *pepN* gene (Tan et al 1992).

### EXAMPLE 1

### Materials and methods

The mature exopeptidase human γ-glutamyl hydrolase was obtained from the full length cDNA (Yao et al. 1996a) cloned in vector pCR2 using the polymerase chain reaction. The vector was provided by The Laboratory of Molecular Diagnostics from the Wadsworth Center, Albany, New York. A PCR product of 885 base-pairs encoding the mature human γ-glutamyl hydrolase was obtained by using the following primers: HGH-f (CATGCCATGGGACCCACGGCGACACCGCCAAG) and HGH-r (GCTCTAGATCAATCAAATATGTAACATTGCTG). The forward primer was extended at the 5' end creating a NcoI restriction site enabling a transcriptional fusion with the inducible nisine promoter in vector pNZ8048 (Kuipers et al.). The use of the mentioned forward primer resulted in slight modification of the mature gene (nucleotides represented in italics). The reverse primer was extended at the 3' end creating a XbaI restriction site enabling a sticky-end ligation in vector pNZ 8048. The newly synthesised vector carrying the mature human γ-glutamyl hydrolase gene is named pNZ7001.

The mature endopeptidase rat γ-glutamyl hydrolase was obtained from the full length cDNA (Yao et al. 1996b) cloned in vector pCR3 using the polymerase chain reaction. The vector was provided by The Laboratory of Molecular Diagnostics from the Wadsworth Center, Albany, New York. A PCR product of 884 basepairs encoding the mature rat γ-glutamyl hydrolase was obtained by using primers: RGH-f (CATGCCATGGGATCCTATGAGCGCGGCTCCAAG) and RGH-r (GCTCTAGATCAGTTAAACATATAAGCTTGCTG). The forward primer was extended at the 5' end creating a NcoI restriction site enabling a transcriptional fusion with an inducible NIS promoter in vector pNZ8048. The use of the mentioned forward primer resulted in slight modification of the mature gene (nucleotides represented in italics). A blunt ended PCR product was cloned in vector PCR blunt (Invitrogen) and transformed into *Escherichia coli.* A partial digested NcoI-KpnI fragment was isolated and ligated into pNZ8048. The newly synthesised vector carrying the mature rat γ-glutamyl hydrolase gene is named pNZ7002.

The vector carrying the mature rat or human γ-glutamyl hydrolase gene was transformed by electroporation to *Lactococcus lactis* strain NZ9000 carrying the nisR and nisK genes on the chromosome. Expression of the mature rat or human γ-glutamyl hydrolase gene was achieved by following the protocol of the NICE system (nisin induced controlled expression) (De Ruyter et al. 1996). Nisin concentrations between 0.1 and 5 ng/ml were used for expression and translation of the human or rat γ-glutamyl hydrolase gene. Western blot analysis was done for human γ-glutamyl hydrolase only using a polyclonal antibody provided by The Laboratory of Molecular Diagnostics from the Wadsworth Center, Albany, New York, USA.

The activity of the mature rat or human γ-glutamyl hydrolase enzyme was determined by growing *L. lactis* strain NZ9000 carrying pNZ7001 or pNZ7002 in 5 ml M17 (Terzaghi and Sandine 1975) supplemented with 0.5% (wt/vol) glucose (GM17) and 10 µg/ml chloramphenicol. GM17 till OD600 was 0.5. At OD600 = 0.5. nisin (1.0 ng/ml) was added to induce expression of the mature rat or human γ-glutamyl hydrolase gene. After two hours of growth, a 20-fold concentrated cell extract (1 ml 0.1M mercaptoethanol, 0.1M Na-PO4 buffer pH 7.0) was made using silica beads and a FP120 fastprep™ cell disrupter (Savant Instruments inc, Holbrook, NY, USA). 500 µl of concentrated cell extract was added to a yeast extract solution (0.5 g/l yeast extract, Difco Laboratories, Detroit, USA), in 20 ml 0.1M Na-PO4 buffer, pH 7.0, 1% ascorbic acid) as a source of polyglutamyl folate derivatives. Incubation at 37°C continued for four hours and samples were taken periodically. The reaction was stopped by heating the samples for 10 minutes at 100°C. The folate concentration was determined by using a *Lactobacillus casei* microbiological assay as described by Horne and Patterson (1988). Negative control experiments were done with strain NZ9000 transformed with pNZ8048 (empty nisin expression cassette).

The activity of the mature rat or human γ-glutamyl hydrolase enzyme in growing lactic acid bacteria was determined by growing *L. lactis* strain NZ9000 carrying pNZ7001 or pNZ7002 in M17 (Terzaghi and Sandine 1975) supplemented with 0.5% (wt/vol) glucose (GM17) and 10 µg/ml chloramphenicol. At OD600 = 0.5. nisin (1.0 ng/ml) was added and growth culture was periodically sampled. Samples were centrifuged until cells could be separated from supernatant. Supernatant was diluted 1:1 with 0.1 M NaAc buffer pH 4.8, 1% ascorbic acid. Cells were washed with 0.1 M NaAc pH 4.8, 1% ascorbic acid and resuspended in the original volume in 0.1 M NaAc buffer pH 4.8, 1% ascorbic acid. All samples were heated to 100°C for ten minutes followed by determination of folate concentration using *Lactobacillus casei* microbiological assay as described by Horne and Patterson (1988). The presence of polyglutamyl folate was analysed by incubating the samples for four hours at 37°C with human plasma (Sigma-Aldrich Chemie, Zwijndrecht, NL) as a source of γ-glutamyl hydrolase followed by folate concentration determination.

### Results

Addition of 1.0 ng/ml of nisin resulted in expression of the expressed human γ-glutamyl hydrolase gene as could be visualised on a western blot using polyclonal antibody (data not shown). The rat γ-glutamyl hydrolase could not be visualised on a western blot because a specific antibody was not available.

### Functional expression of human gamma-glutamyl hydrolase monitored in vitro

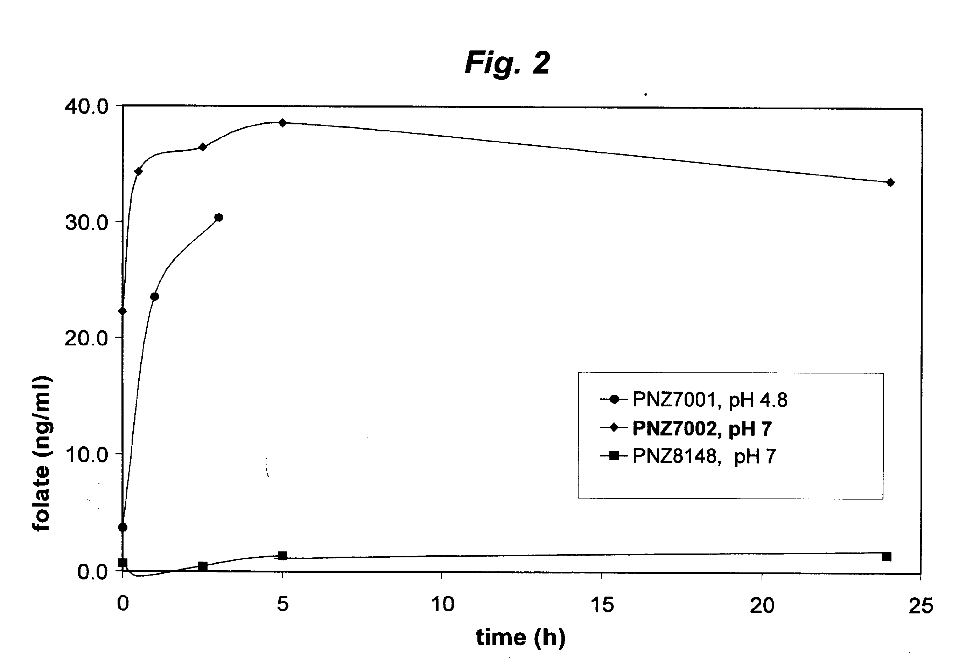

The microbiological folate assay detects mainly folate derivatives with three or less glutamate residues. Growth response of the detection micro-organism to longer folate chains (n>3) decreases markedly in proportion to chain length (Tamura et al. 1972). Consequently, in a source which contains polyglutamyl folates with more than three glutamyl residues, as for instance yeast extract that predominantly contains heptaglutamyl folates (Bassett et al. 1976), folate can only be detected with the microbiological assay when the polyglutamyl tails have been removed. Therefore, yeast extract was used to test the functional expression of human γ-glutamyl hydrolase in *Lactococcus lactis.* The addition of a cell extract of *L. lactis* NZ9000 carrying pNZ7001 or pNZ7002 induced with nisin resulted in deconjugation of polyglutamyl folate as could be visualised by the microbiological assay. The addition of a cell extract of *L. lactis* NZ9000 carrying pNZ8048 (empty nisin expression cassette) did not show any deconjugating activity (Figure 2).

### Intracellular expression of gamma-glutamyl hydrolase monitored in vivo

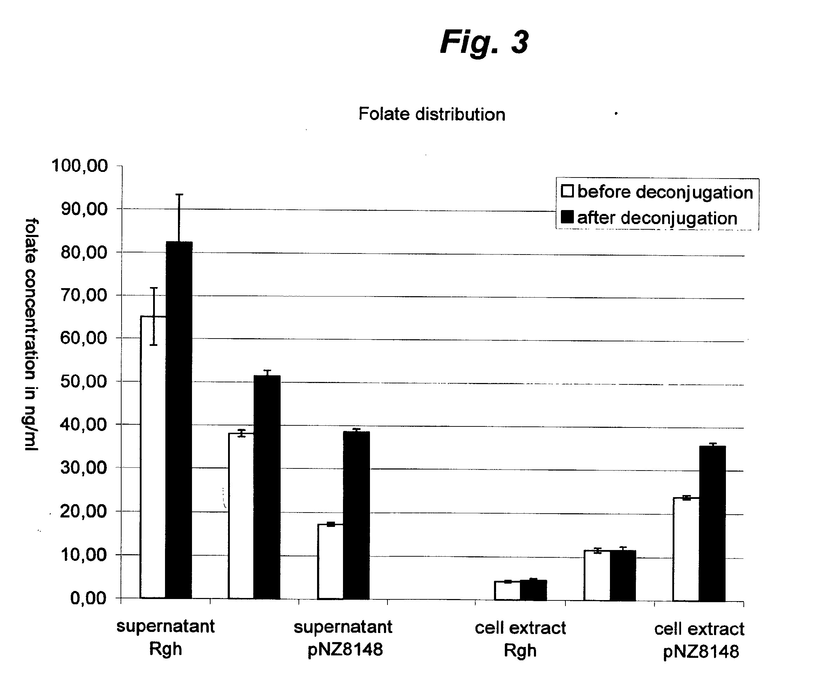

Strain NZ9000 carrying pNZ7001 or pNZ7002 or pNZ8048 (empty nisin expression cassette) showed identical growth characteristics. Induction with nisin at OD600 is 0.5 did not affect growth rate (results not shown). After induction with nisin the folate concentration was measured in the supernatant and in the cell extracts of the strains. *L. lactis* strains NZ9000 carrying pNZ7001 or pNZ7002 are characterised by an increase in extracellular folate concentration during growth. The extracellular folate concentration in *L. lactis* strain NZ9000 carrying pNZ8048 remains constant during growth. The intracellular folate concentration of the strains expressing γ-glutamyl hydrolases does not increase during growth, contrary to the intracellular folate concentration in the control strain. Figure 3 shows the intra- and extracellular folate distribution two hours after induction with nisin. Strain NZ9000-pNZ7002 has the highest extracellular folate concentration probably due to the endopeptidase activity of rat γ-glutamyl hydrolase reducing immediately polyglutamyl folates to monoglutamyl folates. The exopeptidase activity of human γ-glutamyl hydrolase reduces polyglutamyl folate via shorter polyglutamyl folates to monoglutamyl folates. The cell extracts were also checked for the presence of intracellularly stored polyglutamyl folate. Folate levels were determined by using the microbiological assay after incubation with human plasma as a source for γ-glutamyl hydrolase. Strains NZ9000-pNZ7001 and pNZ7002 did not contain polyglutamyl folate and the concentration of folate did not change during growth. The intracellular folate concentration in strain NZ9000-pNZ8048 partly consisted of polyglutamyl folates, see Figure 3. The increase in extracellular folate concentration in the fermentation broth of the strains after deconjugation is generally caused by the presence of polyglutamyl folate from yeast extract which is present in GM17 bacterial growth medium. It can be concluded from the results that the enzymatic activity of the expressed γ-glutamyl hydrolases deconjugate the intracellular polyglutamyl folate resulting in a decreased retention of folate derivatives in the cell resulting in an increase of the extracellular folate concentration.

For information on the DNA sequence of the genes coding for human and rat γ-glutamyl hydrolase, see the reference of YAO et al, 1996 a and b.

### EXAMPLE 2

### Materials and methods

The gene *gch* encoding GTP cyclohydrolase was identified by sequence analysis and homology comparison of part of the genome of *L. lactis* strain MG1363 flanked by the genes *dhfr* encoding dihydrofolate reductase and *dhom* encoding homoserine dehydrogenase with non-redundant genome databases. The sequence was obtained by amplifying a genomic region using primers *dhfrF* (GGAAT TCCAT GGTTA TTGGA ATATG GGCAG AAG) and *dhomR* (CGATC CCGGG AAGCC CTGTG CCACT GTCCA A). The primers were derived from sequence information provided in genbank acc. no. X60681 and X96988 respectively. Homology search suggested that part of the amplified fragment (total length approx. 8 kb) contained the sequence information for GTP cyclohydrolase and 2-amino-4-hydroxy-6-hydroxymethyldihydropteridine pyrophosphokinase. A PCR product obtained by using primers hppk-f2 (CATGC CATGG GGCAA ACAAC TTATT TAAGC ATGGG) and gch-r3 (GGGGT ACCGA TTCTT GATTA AGTTC TAAG) comprising a potential start codon of 2-amino-4-hydroxy-6-hydroxymethyldihydropteridine pyro-phosphokinase and a potential stop codon of GTP cyclohydrolase was cloned into the NcoI and KpnI site of pNZ8048 (Kuipers et al.) generating plasmid pNZ7003. The forward primer was extended at the 5' end, creating an NcoI restriction site enabling a translational fusion with the inducible nisine promoter in vector pNZ8048. The use of the mentioned forward primer resulted in slight modification of the original *gch* gene; an extra glycine was introduced after Met-1 to leave the next amino acid unaltered. The reverse primer was extended at the 3' end creating a KpnI restriction site enabling a sticky-end ligation in vector pNZ8048. The vector was introduced into *L. lactis* strain NZ9000 (De Ruyter et al. 1996) by electroporation creating *L. lactis* strain NZ9000-pNZ7003. Strain NZ9000 expressed the *nisRK* regulatory genes stably integrated at the *pepN* locus. Expression of *gch* was regulated from the nisin promoter by the addition of nisin into the fermentation broth (De Ruyter et al. 1996). Restriction, ligation and transformation were done by following standard protocols as outlined by Sambrook et al.

### Determination of enzymatic activity of GTP cyclohydrolase

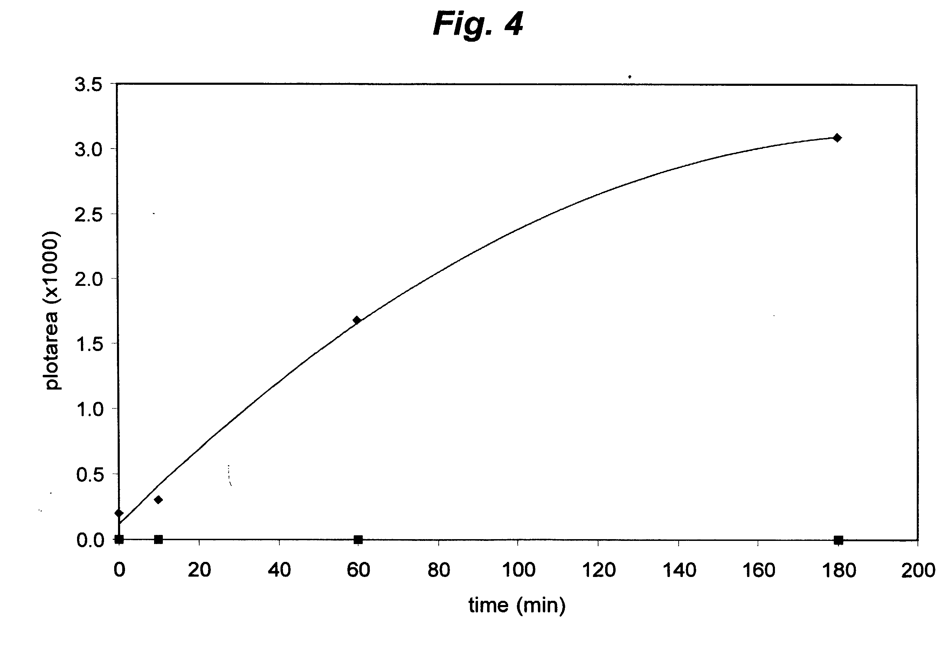

An enzymatic assay to determine whether GTP cyclohydrolase is actively expressed in *L. lactis* was performed. Cell free extract of NZ9000-pNZ7003was used as the source of enzyme and commercial available GTP was used as a substrate. A modified protocol essentially described by Saizieu et al. (1995) was used for the activity assay of GTP cyclohydrolase. *L. lactis* strain NZ9000 carrying pNZ7003 was grown in M17 medium (Terzaghi and Sandine 1975) supplemented with 0.5% (wt/vol) glucose (GM17) and 10 µg/ml chloramphenicol. At OD600 of 0.5, nisin (2 ng/ml) was added. At OD600 of 2.5 cells from a 25 ml culture were harvested and dissolved in 1 ml of 0.1 M sodium phosphate buffer pH 6.5. The intracellular content was released using silica beads and an FP120 fastprep™ cell disrupter (Savant Instruments Inc., Holbrook, NY, USA). The GTP cyclohydrolase activity was determined at 30°C in 100 mM Tris/HCl pH 8, 100 mM KCl, 1 mM EDTA pH 8, 50 iM GTP and 2% cell free extract. Time samples were heated at 100°C for 10 minutes to inactivate the enzyme. The GTP consumption and the product formation were analysed by HPLC. Samples (25 µl) were applied on a 8 **µ** 1000 Å PL-SAX column (Polymer Laboratories) at a flow rate of 1.2 ml/min with a running buffer containing 50 mM phosphate, 100 mM NaCl, pH 6.5. The GTP consumption was monitored by UV absorption at 254 nm (data not shown), the reaction product was detected fluorometrically; excitation at 365 nm, emission at 446 nm. (See Figure 4).

### Overexpression of gch

The effect on folate production by overexpression of the gch encoding GTP cyclohydrolase and 2-amino-4-hydroxy-6-hydroxymethyldihydropteridine pyro-phosphokinase in growing lactic acid bacteria was determined by growing *Lactococcus lactis* strain NZ9000 carrying pNZ7003 in M17 medium (Terzaghi and Sandine, 1975) supplemented with 0.5% (wt/vol) glucose (GM17) and 10 µg/ml chloramphenicol. At OD600 of 0.5, nisin (2 ng/ml) was added. At OD600 of 2.5 cell culture was centrifuged until cells could be separated from supernatant. Supernatant was diluted 1:1 with 0.1 M NaAc buffer pH 4.8, 1% ascorbic acid. Cells were washed with 0.1 M NaAc pH 4.8, 1% ascorbic acid and resuspended in the original volume in 0.1 M NaAc buffer pH 4.8, 1% ascorbic acid. Samples were heated to 100°C for ten minutes followed by determination of folate concentration using *Lactobacillus casei* microbiological assay as described by Horne and Patterson (1988). The presence of polyglutamyl folate was analysed by incubating the samples for four hours at 37°C with human plasma (Sigma-Aldrich Chemie, Zwijndrecht, NL) as a source of γ-glutamyl hydrolase followed by determination of folate concentration using *Lactobacillus casei* microbiological assay.

### Results

The enzymatic activity assay for GTP cyclohydrolase resulted in an increase of a reaction product in time upon addition of GTP (Figure 4). This reaction product had similar chromatographic behaviour as reported before (retention time 9.52 min) [Saizieu et al. (1995)] and was assigned to be dihydroneopterin triphosphate, the main product of GTP cyclohydrolase. The reaction mixture contained an excess of GTP. Decrease in GTP concentration (data not shown) and increase in dihydroneopterin triphosphate was measured only with cell free extract of NZ9000-pNZ7003. Control reactions containing cell free extracts of strain NZ9000-pNZ8048 did not show a significant decrease in GTP, nor formation of products of GTP-conversion.

A qualitative protein analysis of a cell extract of NZ9000-pNZ7003 induced with nisin using SDS-PAGE showed the overproduction of a protein of 40 kD compared to control strain and NZ9000-pNZ8048 (data not shown).

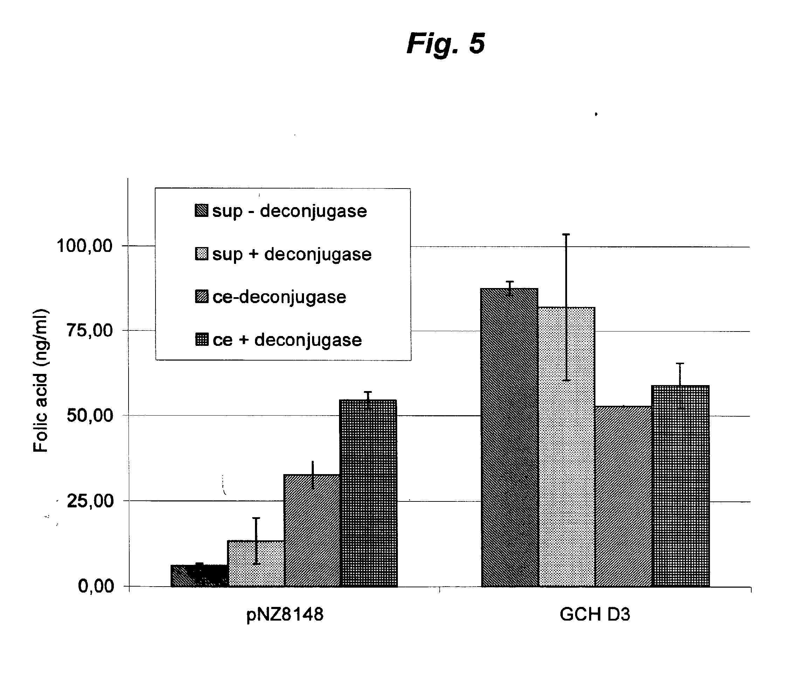

The induction with nisin of growing cells of strain NZ9000-pNZ7003 showed an increase in intra- and extracellular folate concentration compared to the control strain NZ9000-pNZ8048 (Figure 5). The total folate production was doubled. Most of the extra produced folate was present in the growth medium; the extracellular folate concentration increased approximately twenty-fold compared to the control strain. The increase in intracellular folate concentration was of minor importance. Deconjugation of the intracellular folate pool showed that only in the control strain the intracellular folate concentration was partly present as polyglutamyl folate. The GTP cyclohydrolase overproducing strain did not produce folate in the polyglutamyl folate form inside the cells or outside the cells.

### Description of the figures

Figure 1. Pathway for folate biosynthesis. Brackets indicate a presumed reaction intermediate. Triphosphate residues are indicated as (P)₃. Figure adapted from Lacks et al., 1995.
Figure 2. Folic acid concentration in yeast extract at optimal pH as determined with a microbiological assay after deconjugation with a cell extract of *L. lactis* NZ9000 induced with nisin and carrying pNZ7001 encoding rat gamma glutamyl hydrolase, or pNZ7002 encoding human gamma-glutamyl hydrolase or pNZ8048 (empty expression cassette ) as a negative control.
Figure 3. Intra- and extracellular distribution of folate two hours after induction with nisin of exponentially grown cells. The folate distribution is shown before and after enzymatic deconjugation with human plasma deconjugation differentiating the folate derivatives with short glutamic acid residues and longer glutamic acid residues (N>3). PNZ8048, RgH and Hgh resemble *L. lactis* strain NZ9000 with plasmid pNZ8048 (empty expression cassette), pNZ7001 (nisA promoter and mature rat gamma glutamyl hydrolase gene) and pNZ7002 (nisA promoter and mature human gamma glutamyl hydrolase gene) respectively. Folate concentration is measured with a microbiological assay.
Figure 4. Overexpression of GTP cyclohydrolase detected by dihydroneopterin triphosphate concentration as analysed by HPLC and fluorescence (excitation at 365 nm, emission at 446 nm). Legend: Cell free extract of strain NZ9000-pNZ7003 containing *gch* (◆) and a control strain containing NZ9000-pNZ8048 (¦).Figure 5. Intra- (ce) and extracellular (sup) folate concentration of *gch* overexpressing strain NZ9000-pNZ7003 and control strain NZ9000-pNZ8048 (containing empty plasmid pNZ8048.). Folate concentration. Folate concentration is measured with a microbiological assay before and after deconjugation with human plasma as an external source of gamma glutamyl hydrolase.

### References

De Saizieu, A., Vankan, P. and Van Loon, A. P. G. M. (1995) Enzymic Characterization of *Bacillus subtilis* GTP Cyclohydrolase I. *Journal of Biochemistry* **306:** 371-377.
Gregory J. F. Chemical and nutritional aspects of folate research: analytical procedures, methods of folate synthesis, stability, and bioavailability of dietary folates. Adv. Food Nutr. Res. 1989; 33:1-101
Home, D.W., Patterson, D. *Lactobacillus casei* microbiological assay of folic acid derivatives in 96 well microtiter plates. Clin. Chem **34,** 2357-2359 (1988)
Huangpu, J., Pak, J.H., Graham, M.C., Rickle, S.A., Graham, J.S. Purification and molecular analysis of an extracellular gamma-glutamyl hydrolase present in young tissues of the soybean plant. Biochem. Biophys. Res. Commun. **226**, 1-6 (1996)
Kuipers O.P., de Ruyter P., Kleerebezem M., and de Vos W. Quorum sensing-controlled gene expression in lactic acid bacteria. 1998; 64:15-21
Lacks, S. A., Greenberg, B. and Lopez, P. (1995) A Cluster of Four Genes Encoding Enzymes for Five Steps in the Folate Biosynthetic Pathway of *Streptococcuus pneumoniae.* Journal of Bacteriology, 66-74.
Leshchinskaya, I.B., Shakirov, E.V., Itskovitch, E.L., Balaban, N.P., Mardano, A.M., Sharipova, M.R., Blagova, E.V., Levdikov, V.M., Kuranova, I.P., Rudenskaya, G.N. and Stepanov, V.M. (1997) Glutamyl endopeptidase of *Bacillus intermedius* strain 3-19. Purification, properties, and crystaiiization. Biochemistry (Mosc) **62**: 903-908.
Rooijen, R.J. van, Gasson, M.J., de Vos, W.M. Characterization of the Lactococcus lactis lactose operon promoter: contribution of flanking sequences and LacR repressor to promoter activity. J. Bacteriol. **174(7),**2273-80 (1992).
Rooijen, R.J. van, de Vos, W.M. Molecular cloning, transcriptional analysis, and nucleotide sequence of lacR, a gene encoding the repressor of the lactose phosphotransferase system of Lactococcus lactis. J Biol Chem. **265(30),**18499-503 (1990).
Rosenberg I. H., Godwin H. A. Inhibition of intestinal gamma-glutamyl carboxypeptidase by yeast nucleic acid: an explanation of variability in utilization of dietary polyglutamyl folate. J. Clin. Investig. 1971.
Ruyter P.G. de, Kuipers O.P., de Vos W.M. Controlled gene expression systems for Lactococcus lactis with the food-grade inducer nisin. Appl. Environ. Microbiol. 1996 Oct;62(10):3662-7.
Seyoum E, Selhub J Properties of food folates determined by stability and susceptibility to intestinal pteroylpolyglutamate hydrolase action. J. Nutr. 1998 Nov;128(11):1956-60
Shane B. Folylpolyglutamate synthesis and role in the regulation of one-carbon metabolism. Vitam Horm. 1989;45:263-335.
Tan, P.S., van Alen-Boerrigter, I.J., Poolman, B., Siezen R.J., de Vos, W.M., Konings, W.N. Characterization of the Lactococcus lactis pepN gene encoding an aminopeptidase homologous to mammalian aminopeptidase N. FEBS Lett. 1992 Jul 13;306(1):9-16.
Terzaghi, B.E. and Sandine W.E. Improved medium for lactic streptococci and their bacteriophages. Appl. Microbiol. Biotechnol. 1975;38:17-22
Yao, R., Schneider, E., Ryan, T.J., Galivan, J. Human gamma-glutamyl hydrolase: cloning and characterization of the enzyme expressed in vitro. Proc. Natl. Acad. Sci. USA. 1996a Sep 17;93(19):10134-8.
Yao, R., Nimec, Z., Ryan, T.J., Galivan, J. Identification, cloning, and sequencing of a cDNA coding for rat gamma-glutamyl hydrolase. J. Biol. Chem. 1996b Apr 12;271(15):8525-8.

## Claims

1. A genetically modified micro-organism, wherein the modification results in an increase of the amount of monoglutamyl folate produced, relative to the amount produced by the unmodified micro-organism.

2. A micro-organism according to claim 1, in which the activity of at least one enzyme involved in monoglutamyl folate synthesis is increased.

3. A micro-organism according to claim 2, in which said enzyme is selected from GTP cyclohydrolase encoded by the *gch* gene and 2-amino-4-hydroxy-6-hydroxymethyl-dihydropteridine pyro-phosphokinase encoded by the *hppk* gene.

4. A micro-organism according to claim 1, in which activity of gamma-glutamyl hydrolase is increased.

5. A micro-organism according to claim 2 or 3, in which said increased enzyme activity is the result of the presence of multiple copies of the corresponding genes.

6. A micro-organism according to claim 2 or 3, in which said increased enzyme activity is the result of the presence a promoter enhancing expression of the corresponding genes.

7. A micro-organism according to any one of claims 1-6, in which the genes coding for said enzyme activities are of animal, in particular mammalian origin.

8. A micro-organism according to any one of claims 1-6, in which the genes coding for said enzyme activities are of plant origin.

9. A micro-organism according to any one of claims 1-6, in which the genes coding for said enzyme activities are of microbial origin, in particular from a lactic acid bacterium or a yeast.

10. A process of producing monoglutamyl folate, comprising culturing a micro-organism according to any one of claims 1-9 and recovering the folates produced.

11. A process of supplying bioavailable folate to the gastrointestinal tract of a mammal comprising administering an effective amount of a micro-organism according to any one of claims 1-9.

12. A food product containing a micro-organism according to any one of claims 1-9.

13. A food product according to claim 12, which is a dairy product.
